# EUROPEAN PATENT APPLICATION

(11) **EP 3 088 803 A1**
(43) Date of publication of application: **02.11.2016**
(21) Application number: 16167006.2
(22) Date of filing: 26.04.2016
(51) Int. Cl.: F23N 5/00

(54) **COMBUSTION OPTIMIZATION SYSTEM AND METHOD**

(30) Priority: 30.04.2015 CN 201510218721
(71) Applicant: General Electric Company, Schenectady, NY 12345 (US)
(72) Inventor: WANG, Honggang, Malta, NY New York 12020 (US); CHEN, Yao, 201203 Shanghai (CN); ZHOU, Yingneng, 2012013 Shanghai (CN); MOYEDA, Daid K., Irvine, CA California 92614 (US)
(74) Representative: Foster, Christopher Michael

(57) **Abstract**

A combustion optimization system is disclosed. The system includes a boiler (1) having a plurality of zonal locations, a sensor grid comprising a plurality of sensors, a sensor validation device (3) and an optimizing controller (4). The plurality of sensors are configured to provide a plurality of sensor signals (S) and the plurality of sensor signals (S) are indicative of measurements of the respective zonal locations. The sensor validation device (3) is configured for receiving the plurality of sensor signals (S) from the plurality of sensors (S) and generating validated sensor signals (Sv) of the respective sensors based on the plurality of received sensor signals and predetermined correlations among the plurality of received sensor signals. The optimizing controller (4) is configured for optimizing at least one operating parameter of the boiler (1) based on the validated sensor signals of the respective sensors. A combustion optimization method is also disclosed.

## Description

### BACKGROUND

This disclosure relates generally to the field of controlling, and more particularly to a combustion optimization system and a combustion optimization method.

Sensors are usually used to measure and gather a variety of data associated with important operating parameters of a system, such as temperature, pressure, gas concentration and the like. Outputs of the sensors will change based on changing conditions in the system. Thus, a typical use of the sensors is to monitor performance of the system so that the performance of the system may be efficiently controlled. Signals from the sensors can be provided for evaluation. Based on the evaluation result, one or more operating parameters of the system will be altered or controlled in order to improve efficiency of the system. Better control of the system is possible when the sensor signal is more accurate, so the accuracy of the sensor signals will play an important role in the control of the system. However, determining whether the sensors are providing accurate data is very difficult when the system operates in a harsh environment, such as a high temperature or a high pressure environment that may damage the sensors. If a sensor that reflects an operational parameter of the system is broken and is not able to supply an accurate signal, the control of the system based on the output of the broken sensor may be less efficient.

For example, in a boiler system, a plurality of sensors are used to determine a combustion control strategy of the boiler system. However, the harsh environment in the boiler system will inevitably make the sensors prone to aging, degradation and failure as time lapses. Thus, signals from these low-performance sensors will not accurately reflect the data of the boiler system, and may also cause improper combustion control. Such improper combustion control may lead to lower combustion efficiency, higher nitrogen oxides and carbon monoxide concentrations, and reduced reliability. Furthermore, such improper combustion control may also lead to increased slagging and increased boiler tube failures, and even lead to catastrophic consequences like furnace fire extinction or explosion.

### BRIEF DESCRIPTION

In one aspect of embodiments of the present invention, a combustion optimization system is provided. The combustion optimization system comprises a boiler having a plurality of zonal locations, a sensor grid comprising a plurality of sensors, a sensor validation device and an optimizing controller. The plurality of sensors are configured to provide a plurality of sensor signals and the plurality of sensor signals are indicative of measurements of the respective zonal locations. The sensor validation device is configured for receiving the plurality of sensor signals from the plurality of sensors and generating validated sensor signals of the respective sensors based on the plurality of received sensor signals and pre-determined correlations among the plurality of received sensor signals. The optimizing controller is configured for optimizing at least one operating parameter of the boiler based on the validated sensor signals of the respective sensors.

In another aspect of embodiments of the present invention, a combustion optimization method is also provided. The combustion optimization method comprises: receiving a plurality of sensor signals from a sensor grid which comprises a plurality of sensors configured to be in communication with a plurality of zonal locations in a boiler; generating validated sensor signals of the respective sensors based on the plurality of received sensor signals and pre-determined correlations among the plurality of received sensor signals; and optimizing at least one operating parameter of the boiler based on the validated sensor signals of the respective sensors.

### DRAWINGS

These and other features, aspects, and advantages of the present disclosure will become better understood when the following detailed description is read with reference to the accompanying drawings in which like characters represent like parts throughout the drawings, wherein:
FIG. 1 is a schematic block diagram of a combustion optimization system in accordance with an embodiment of the present invention;
FIG. 2 is a schematic diagram of a boiler of the combustion optimization system of FIG. 1;
FIG. 3 is a schematic block diagram of a sensor validation device in accordance with an embodiment of the present invention;
FIG. 4 is a schematic block diagram of a sensor validation device in accordance with another embodiment of the present invention;
FIG. 5 is a flow chart of a combustion optimization method in accordance with an embodiment of the present invention; and
FIG. 6 illustrates steps how to determine overall sensor health confidence values of respective sensors of FIG. 5.

### DETAILED DESCRIPTION

Embodiments of the present disclosure will be described herein below with reference to the accompanying drawings. In the following description, well-known functions or constructions are not described in detail to avoid obscuring the disclosure in unnecessary detail.

Unless defined otherwise, technical and scientific terms used herein have the same meaning as is commonly understood by one of ordinary skill in the art to which this disclosure belongs. The terms "first", "second", and the like, as used herein do not denote any order, quantity, or importance, but rather are used to distinguish one element from another. Also, the terms "a" and "an" do not denote a limitation of quantity, but rather denote the presence of at least one of the referenced items. The term "or" is meant to be inclusive and mean either or all of the listed items. The use of "including," "comprising" or "having" and variations thereof herein are meant to encompass the items listed thereafter and equivalents thereof as well as additional items. Further, the phrase "based on" means "based at least in part on".

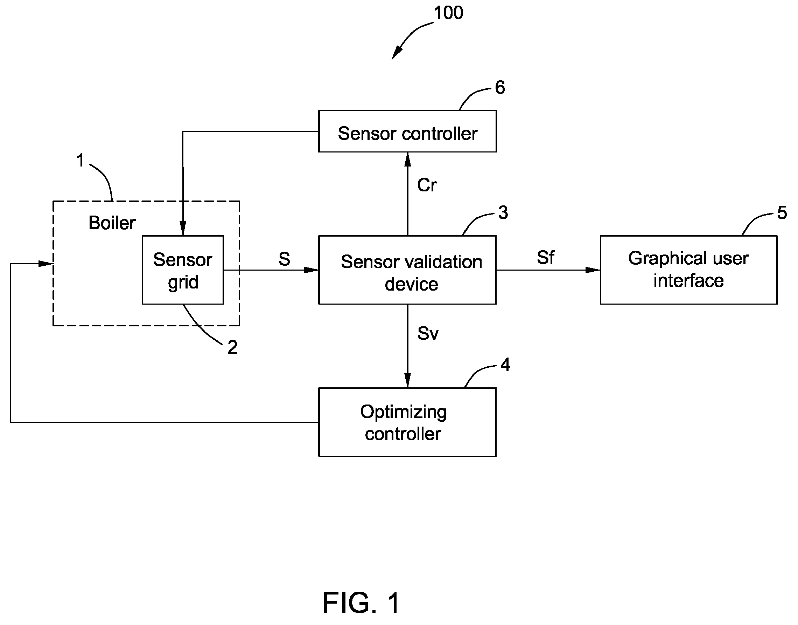

FIG. 1 illustrates a schematic block diagram of a combustion optimization system in accordance with an embodiment of the present invention. As shown in FIG. 1, the combustion optimization system 100 in accordance with an embodiment of the present invention comprises a boiler 1, a sensor grid 2, a sensor validation device 3 and an optimizing controller 4.

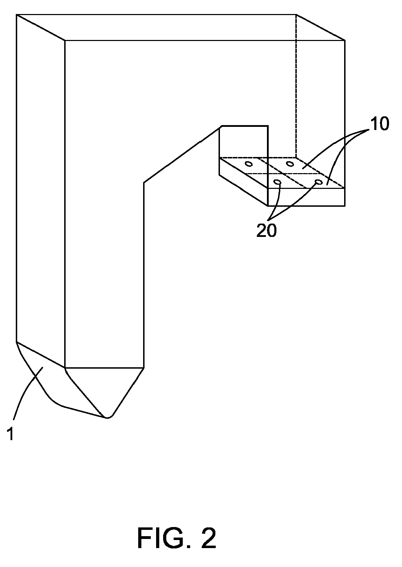

FIG. 2 illustrates a schematic diagram of the boiler 1. With reference to FIG. 2, the boiler 1 has a plurality of zonal locations 10 which are schematically shown to be a 2×2 matrix. In FIG. 2, as an example, the plurality of zonal locations 10 are shown to be at a back pass of the boiler 1. However, the plurality of zonal locations 10 may be located in any position of the boiler 1 as long as data coming from the plurality of zonal locations 10 can reflect operating status of the boiler 1. The sensor grid 2 comprises a plurality of sensors 20 which are schematically shown to be a 2×2 matrix. The plurality of sensors 20 are in communication with the plurality of zonal locations 10. In FIG. 2, as an example, the plurality of sensors 20 are shown to be respectively situated in the plurality of zonal locations 10. However, the locations of the plurality of sensors 20 of the present invention should be not limited hereinto. In combination with FIG. 1, the plurality of sensors 20 are configured to provide a plurality of sensor signals S and the plurality of sensor signals S are indicative of measurements of the respective zonal locations 10 of the boiler 1. When the plurality of sensors 20 are healthy, the sensor signals S from the healthy sensors 20 can accurately reflect data of the respective zonal locations 10. But if the sensors 20 become faulty, the sensor signals S from the faulty sensors 20 will not accurately reflect data of the respective zonal locations 10. Thus, the combustion optimization system 100 of the present invention provides the sensor validation device 3 of FIG. 1.

Returning now to FIG. 1, the sensor validation device 3 receives the plurality of sensor signals S from the plurality of sensors 20 of the sensor grid 2 and generates validated sensor signals Sv of the respective sensors 20 based on the plurality of received sensor signals S and pre-determined correlations among the plurality of received sensor signals S. The sensor validation device 3 of the present invention will generate the validated sensor signals Sv of the respective sensors 20 regardless of the healthy sensors 20 or the faulty sensors 20, thereby securing normal operation of the combustion optimization system 100 and reducing erroneous combustion control. The optimizing controller 4 optimizes at least one operating parameter of the boiler 1 based on the validated sensor signals Sv of the respective sensors 20, resulting in improving the combustion control strategy of the combustion optimization system 100 of the present invention.

As shown in FIG. 1, in one embodiment, the combustion optimization system 100 of the present invention may further include a graphical user interface 5. The graphical user interface 5 is connected with the sensor validation device 3. When the sensor validation device 3 determines that at least one of the plurality of sensors 20 is faulty, the sensor validation device 3 is also configured to generate a fault warning signal Sf to the graphical user interface 5, thereby enabling timely maintenance and recovery of the combustion optimization system 100 and avoiding progressive damage and equipment downtime.

In another embodiment, the combustion optimization system 100 of the present invention may further include a sensor controller 6 for controlling the plurality of sensors 20. The sensor controller 6 is connected with the sensor validation device 3. When the sensor validation device 3 determines that at least one of the plurality of sensors 20 is faulty and if the fault is of a type that may be compensated or repaired via a sensor control signal, the sensor validation device 3 is also configured to generate a repairing command Cr to the sensor controller 6.

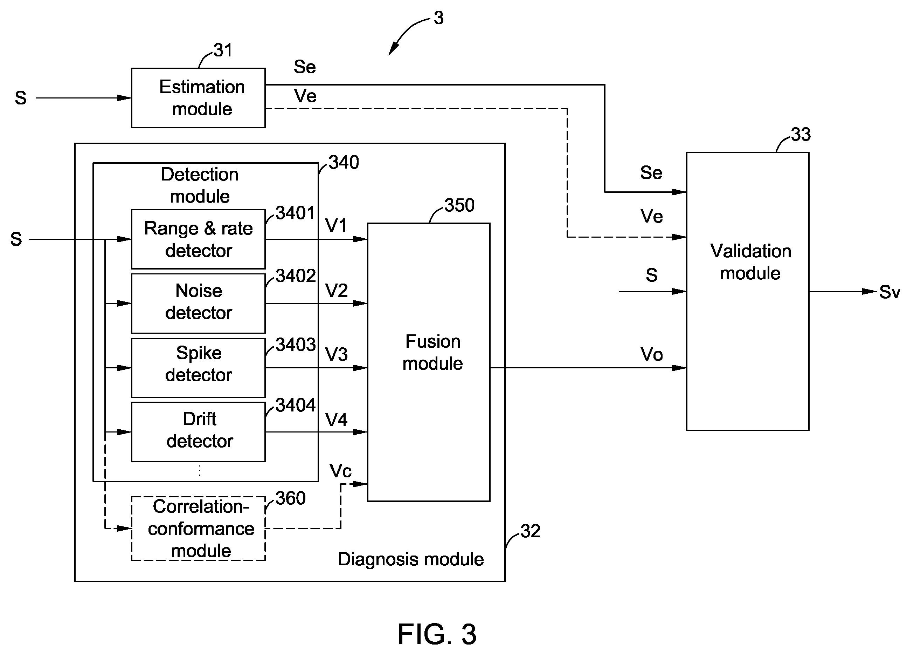

FIG. 3 illustrates a schematic block diagram of the sensor validation device 3 in accordance with an embodiment of the present invention. As shown in FIG. 3, the sensor validation device 3 includes an estimation module 31. The estimation module 31 receives the plurality of sensor signals S and generates estimated sensor signals Se of the respective sensors 20 based on the plurality of received sensor signals S. The estimated sensor signals Se may be generated based on the pre-determined correlations among the plurality of received sensor signals S. For example, the estimated sensor signals Se may be generated based on spatial correlations among the plurality of received sensor signals S. The sensor validation device 3 generates the validated sensor signals Sv of the respective sensors 20 based on the respective received sensor signals S and the respective estimated sensor signals Se.

Continuing to refer to FIG. 3, the sensor validation device 3 further comprises a diagnosis module 32 and a validation module 33. The diagnosis module 32 receives the plurality of sensor signals S and determines overall sensor health confidence values Vo of the respective sensors 20. The overall sensor health confidence values Vo are indicative of reliability of the respective sensors 20. The validation module 33 then generates the validated sensor signals Sv of the respective sensors 20 based on the respective received sensor signals S, the respective estimated sensor signals Se and the respective overall sensor health confidence values Vo.

In one embodiment, the diagnosis module 32 comprises a detection module 340 and a fusion module 350. The detection module 340 is configured to receive the plurality of sensor signals S, detect fault types of the respective sensors 20 and generate fault type confidence values, for example, V1, V2, V3, V4 of the respective sensors 20. The fault type confidence values V1, V2, V3, V4 are indicative of fault levels of the respective fault types. The fusion module 350 is configured to fuse the generated fault type confidence values V1, V2, V3, V4 of the respective sensors 20 to generate the overall sensor health confidence values Vo of the respective sensors 20.

In an embodiment of the present invention, the fault types of the sensor 20 may include, but not limited to range and rate, noise, spike and drift. The spike of the sensor 20 may be defined as the unexpected instantaneous change of the sensor reading when compared to the recent history of the sensor reading with all operating conditions of the system remaining unchanged. The drift of the sensor 20 may be defined as the deviation of the sensor reading from its predicted or expected value. The fault types above are shown only as an example. However, the fault types of the sensor 20 of the present invention should be not limited hereinto. Corresponding to these fault types, as shown in FIG. 3, the detection module 340 of the present invention may include, but not limited to a range and rate detector 3401, a noise detector 3402, a spike detector 3403 and a drift detector 3404. Similarly, the detection module 340 of the present invention should be not limited hereinto and may include other types of detectors. In one embodiment, the number of the detectors that the detection module 340 includes may be correspondingly adjusted when the fault types of the sensor 20 that is to be detected change.

In detail, the range and rate detector 3401 detects range and rate faults of the respective sensors 20 and then generates range and rate fault confidence values V1 of the respective sensors 20. The range and rate fault confidence value V1 is indicative of fault level of the range and rate fault. The noise detector 3402 detects noise faults of the respective sensors 20 and then generates noise fault confidence values V2 of the respective sensors 20. The noise fault confidence value V2 is indicative of fault level of the noise fault. The spike detector 3403 detects spike faults of the respective sensors 20 and then generates spike fault confidence values V3 of the respective sensors 20. The spike fault confidence value V3 is indicative of fault level of the spike fault. The drift detector 3404 detects drift faults of the respective sensors 20 and then generates drift fault confidence values V4 of the respective sensors 20. The drift fault confidence value V4 is indicative of fault level of the drift fault.

In another embodiment of the present invention, the diagnosis module 32 may further comprise a correlation-conformance module 360. The correlation-conformance module 360 receives the plurality of sensor signals S from the plurality of sensors 20 and generates correlation-conformance indexes Vc of the respective sensors 20 based on the pre-determined correlations among the plurality of received sensor signals S. The correlation-conformance indexes Vc of the respective sensors 20 are indicative of fault levels of the respective sensors 20. For example, the pre-determined correlations among the plurality of received sensor signals S may comprise spatial correlations among the plurality of received sensor signals S. In one embodiment, the fusion module 350 further fuses the generated fault type confidence values V1, V2, V3, V4 of the respective sensors 20 and the correlation-conformance indexes Vc of the respective sensors 20 so as to generate the overall sensor health confidence values Vo of the respective sensors 20.

With reference to FIG. 3, in another embodiment, the estimation module 31 of the present invention may also generate an estimation module confidence value Ve. The estimation module confidence value Ve is indicative of reliability of the estimated sensor signals Se. In one embodiment, the validation module 33 generates the validated sensor signals Sv of the respective sensors 20 further based on the estimation module confidence value Ve.

The combustion optimization system 100 of the present invention can generate the validated sensor signals Sv of the respective sensors 20 based on the plurality of received sensor signals S and the pre-determined correlations among the plurality of received sensor signals S regardless of the healthy sensors 20 or the faulty sensors 20, so the combustion optimization system 100 of the present invention can reduce erroneous operation, improve service factor, optimize the combustion strategy of the system, increase system robustness and reduce economic loss due to sensor fault.

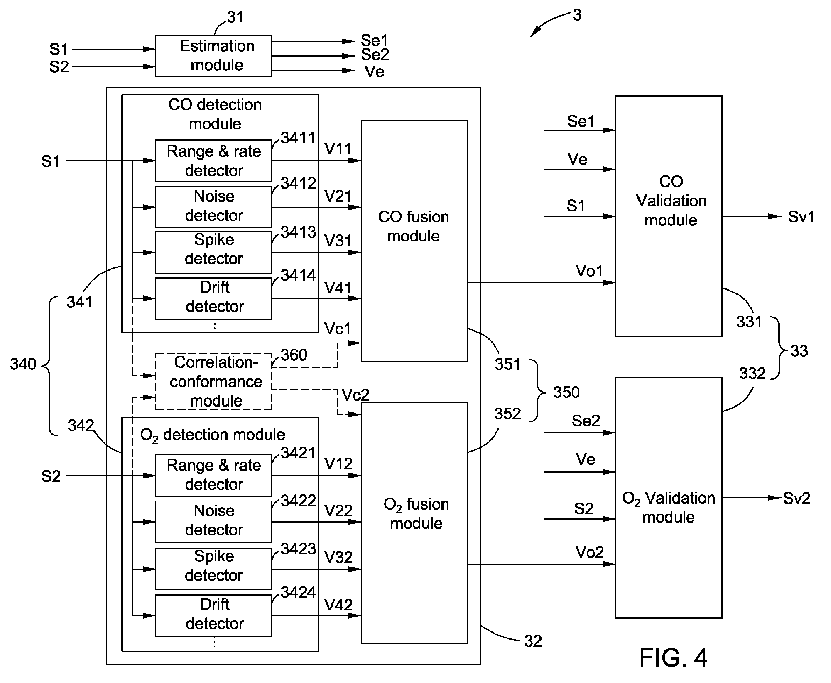

In another embodiment of the combustion optimization system 100 of the present invention, the plurality of sensors 20 may comprise a plurality of CO sensors (not labeled) and a plurality of O₂ sensors (not labeled). The plurality of CO sensors are configured to provide a plurality of CO sensor signals S1 (as shown in FIG. 4) which are indicative of CO concentrations passing through the respective zonal locations 10. The plurality of O₂ sensors are configured to provide a plurality of O₂ sensor signals S2 (as shown in FIG. 4) which are indicative of O₂ concentrations passing through the respective zonal locations 10.

FIG. 4 illustrates a schematic block diagram of a sensor validation device 3 in accordance with another embodiment of the present invention. Correspondingly, the estimation module 31 is configured for respectively receiving the plurality of CO sensor signals S1 and the plurality of O₂ sensor signals S2, and generates estimated CO sensor signals Se1 of the respective CO sensors based on the plurality of received CO sensor signals S 1 and estimated O₂ sensor signals Se2 of the respective O₂ sensors based on the plurality of received O₂ sensor signals S2. For example, the estimated CO sensor signals Se1 of the respective CO sensors may be generated based on spatial correlations among the plurality of received CO sensor signals S1, and the estimated O₂ sensor signals Se2 of the respective O₂ sensors may be generated based on spatial correlations among the plurality of received O₂ sensor signals S2. In the diagnosis module 32, the detection module 340 comprises a CO detection module 341 and an O₂ detection module 342, and the fusion module 350 comprises a CO fusion module 351 and an O₂ fusion module 352. The validation module 33 comprises a CO validation module 331 and an O₂ validation module 332.

The CO detection module 341 is configured to receive the plurality of CO sensor signals S1, detect fault types of the respective CO sensors and generate fault type confidence values, for example, V11, V21, V31, V41 of the respective CO sensors. As an example, the CO detection module 341 may include, but not limited to a range and rate detector 3411, a noise detector 3412, a spike detector 3413 and a drift detector 3414. In the CO detection module 341, the range and rate detector 3411 detects range and rate faults of the respective CO sensors and then generates range and rate fault confidence values V11 of the respective CO sensors, the noise detector 3412 detects noise faults of the respective CO sensors and then generates noise fault confidence values V21 of the respective CO sensors, the spike detector 3413 detects spike faults of the respective CO sensors and then generates spike fault confidence values V31 of the respective CO sensors, and the drift detector 3414 detects drift faults of the respective CO sensors and then generates drift fault confidence values V41 of the respective CO sensors.

Similarly, the O₂ detection module 342 is configured to receive the plurality of O₂ sensor signals S2, detect fault types of the respective O₂ sensors and generate fault type confidence values, for example, V12, V22, V32, V42 of the respective O₂ sensors. As an example, the O₂ detection module 342 may include, but not limited to a range and rate detector 3421, a noise detector 3422, a spike detector 3423 and a drift detector 3424. In the O₂ detection module 342, the range and rate detector 3421 detects range and rate faults of the respective O₂ sensors and then generates range and rate fault confidence values V12 of the respective O₂ sensors, the noise detector 3422 detects noise faults of the respective O₂ sensors and then generates noise fault confidence values V22 of the respective O₂ sensors, the spike detector 3423 detects spike faults of the respective O₂ sensors and then generates spike fault confidence values V32 of the respective O₂ sensors, and the drift detector 3424 detects drift faults of the respective O₂ sensors and then generates drift fault confidence values V42 of the respective O₂ sensors.

The CO fusion module 351 is configured to fuse the generated fault type confidence values V11, V21, V31, V41 of the respective CO sensors to generate overall CO sensor health confidence values Vo1 of the respective CO sensors. For example, the range and rate fault confidence values V11, the noise fault confidence values V21, the spike fault confidence values V31 and the drift fault confidence values V41 of the respective CO sensors are fused by the CO fusion module 351, and the overall CO sensor health confidence values Vo1 of the respective CO sensors are then generated.

Similarly, the O₂ fusion module 352 is configured to fuse the generated fault type confidence values V12, V22, V32, V42 of the respective O₂ sensors to generate overall O₂ sensor health confidence values Vo2 of the respective O₂ sensors. For example, the range and rate fault confidence values V12, the noise fault confidence values V22, the spike fault confidence values V32 and the drift fault confidence values V42 of the respective O₂ sensors are fused by the O₂ fusion module 352, and the overall O₂ sensor health confidence values Vo2 of the respective O₂ sensors are then generated.

In the embodiment of FIG. 4, the diagnosis module 32 of the present invention may further comprise a correlation-conformance module 360. The correlation-conformance module 360 is configured for respectively receiving the plurality of CO sensor signals S1 and the plurality of O₂ sensor signals S2, and generating CO correlation-conformance indexes Vc1 of the respective CO sensors and O₂ correlation-conformance indexes Vc2 of the respective O₂ sensors based on the pre-determined correlations among the plurality of CO sensor signals S1 and the plurality of O₂ sensor signals S2. For example, the pre-determined correlations may further include correlations of physical characteristics between the respective CO sensor signals S1 and the respective O₂ sensor signals S2. In this embodiment, the CO correlation-conformance indexes Vc1 of the respective CO sensors and the O₂ correlation-conformance indexes Vc2 of the respective O₂ sensors are generated based on the correlations of physical characteristics between the respective CO sensor signals S 1 and the respective O₂ sensor signals S2.

When the diagnosis module 32 includes the correlation-conformance module 360, the CO fusion module 351 further fuses the generated fault type confidence values (for example, the range and rate fault confidence values, the noise fault confidence values, the spike fault confidence values and the drift fault confidence values) V11, V21, V31, V41 of the respective CO sensors and the CO correlation-conformance indexes Vc1 of the respective CO sensors to generate the overall CO sensor health confidence values Vo1 of the respective CO sensors, and the O₂ fusion module 352 further fuses the generated fault type confidence values (for example, the range and rate fault confidence values, the noise fault confidence values, the spike fault confidence values and the drift fault confidence values) V12, V22, V32, V42 of the respective O₂ sensors and the O₂ correlation-conformance indexes Vc2 of the respective O₂ sensors to generate the overall O₂ sensor health confidence values Vo2 of the respective O₂ sensors.

The CO validation module 331 is configured in this embodiment to generate validated CO sensor signals Sv1 of the respective CO sensors based on the respective received CO sensor signals S1, the respective estimated CO sensor signals Se1, and the respective overall CO sensor health confidence values Vo1. Similarly, the O₂ validation module 332 is configured in this embodiment to generate validated O₂ sensor signals Sv2 of the respective O₂ sensors based on the respective received O₂ sensor signals S2, the respective estimated O₂ sensor signals Se2, and the respective overall O₂ sensor health confidence values Vo2.

The estimation module 31 may further generate an estimation module confidence value Ve. The estimation module confidence value Ve is indicative of reliability of the estimated CO and O₂ sensor signals Se1 and Se2. In one embodiment, the CO validation module 331 generates the validated CO sensor signals Sv1 based on the respective received CO sensor signals S1, the respective estimated CO sensor signals Se1, the respective overall CO sensor health confidence values Vo1 and the estimation module confidence value Ve, and the O₂ validation module 332 generates the validated O₂ sensor signals Sv2 based on the respective received O₂ sensor signals S2, the respective estimated O₂ sensor signals Se2, the respective overall O₂ sensor health confidence values Vo2 and the estimation module confidence value Ve.

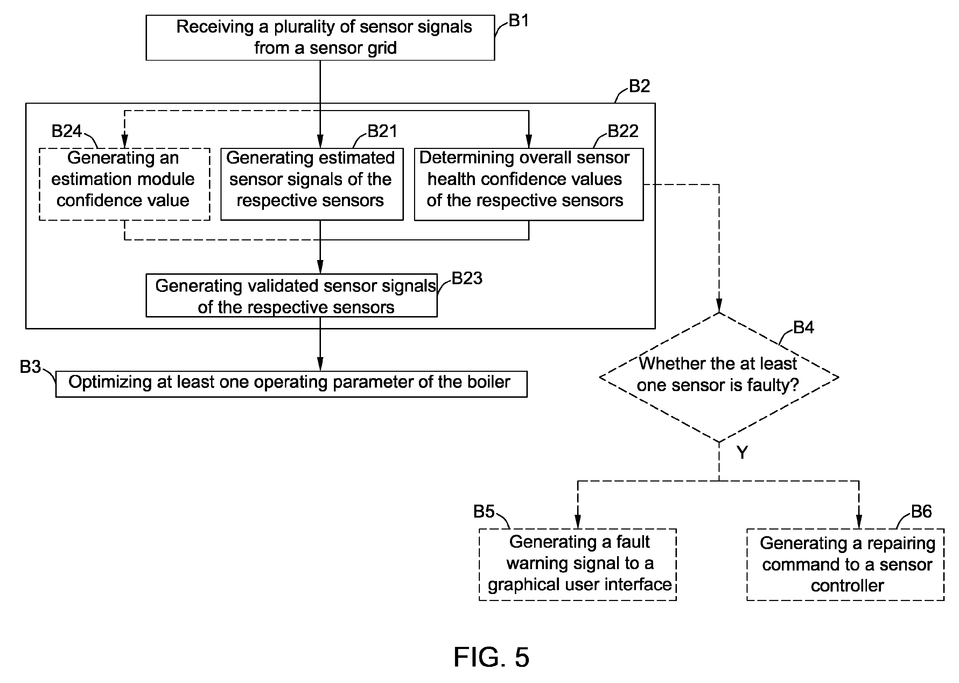

FIG. 5 illustrates a flow chart of a combustion optimization method in accordance with an embodiment of the present invention. As shown in FIG. 5, the combustion optimization method in accordance with an embodiment of the present invention may include the steps as following:
In block B1, a plurality of sensor signals S from a sensor grid 2 (shown in FIG. 1) are received. The sensor grid 2 comprises a plurality of sensors 20 which are configured to be in communication with a plurality of zonal locations 10 in a boiler 1 (shown in FIG. 2).

In block B2, validated sensor signals Sv of the respective sensors 20 of the sensor grid 2 are generated based on the plurality of received sensor signals S and pre-determined correlations among the plurality of received sensor signals S. For example, when the plurality of sensors 20 include the same type of sensors 20, the pre-determined correlations among the plurality of sensor signals S may include spatial correlations among the plurality of sensor signals S. Additionally or alternatively, the pre-determined correlations among the plurality of sensor signals S may also include time correlations among the plurality of sensor signals S. When the plurality of sensors 20 include more than two types of sensors 20, the pre-determined correlations among the plurality of sensor signals S may further include correlations of physical characteristics between the respective sensor signals S1, S2 of different types.

In an embodiment, the step B2 may include the steps as following:
In block B21, estimated sensor signals Se of the respective sensors 20 are generated by an estimation module 31 based on the plurality of received sensor signals S. The estimated sensor signals Se may be generated based on the pre-determined correlations among the plurality of received sensor signals S. For example, the pre-determined correlations may include spatial correlations among the plurality of received sensor signals S.
In block B22, overall sensor health confidence values Vo of the respective sensors 20 are determined based on the plurality of received sensor signals S. The overall sensor health confidence values Vo of the respective sensors 20 are indicative of reliability of the respective sensors 20.

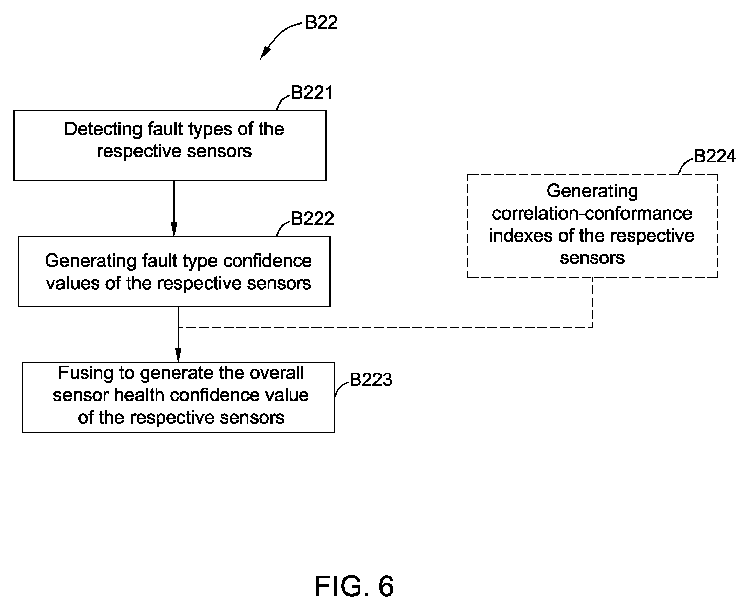

FIG. 6 illustrates steps how to determine the overall sensor health confidence values Vo of the respective sensors 20 of FIG. 5. With reference to FIG. 6, in an embodiment, the step B22 may comprise the steps as following:
In block B221, fault types of the respective sensors 20 such as range and rate fault, noise fault, spike fault, drift fault and etc. are detected based on the plurality of received sensor signals S.
In block B222, fault type confidence values V1, V2, V3, V4 of the respective sensors 20 such as range and rate fault confidence value, noise fault confidence value, spike fault confidence value, drift fault confidence value and etc. are generated. The fault type confidence values V1, V2, V3, V4 are indicative of fault levels of the respective fault types.
In block B223, the generated fault type confidence values V1, V2, V3, V4 of the respective sensors 20 are fused to generate the overall sensor health confidence value Vo of the respective sensors 20.

In another embodiment, the step B22 may further comprise the following step:
In block B224, correlation-conformance indexes Vc of the respective sensors 20 are generated based on the pre-determined correlations among the plurality of received sensor signals S. The correlation-conformance indexes Vc of the respective sensors 20 are indicative of fault levels of the respective sensors 20. In one embodiment, when the plurality of sensors 20 include the same type of sensors, the correlation-conformance indexes Vc of the respective sensors 20 may be generated based on spatial correlations among the plurality of received sensor signals S. In another embodiment, when the plurality of sensors 20 include more than two types of sensors, for example, first sensors and second sensors, correspondingly the plurality of sensor signals S include a plurality of first sensor signals S1 and a plurality of second sensor signals S2, the correlation-conformance indexes Vc of the respective sensors 20 may be generated based on correlations of physical characteristics between the respective first sensor signals S 1 and the respective second sensor signals S2. In one embodiment, the step B223 may comprise: fusing the generated fault type confidence values V1, V2, V3, V4 of the respective sensors 20 and the correlation-conformance indexes Vc of the respective sensors 20 to generate the overall sensor health confidence values Vo of the respective sensors 20.

Returning now to FIG. 5, in block B23, the validated sensor signals Sv of the respective sensors 20 are generated based on the respective received sensor signals S, the respective estimated sensor signals Se, and the respective overall sensor health confidence values Vo.

In another embodiment, the step B2 may further include the following step:
In block B24, an estimation module confidence value Ve is generated. The estimation module confidence value Ve is indicative of reliability of the estimated sensor signals Se. In one embodiment, the step B23 may comprise: generating the validated sensor signals Sv based on the respective received sensor signals S, the respective estimated sensor signals Se, the respective overall sensor health confidence values Vo and the estimation module confidence value Ve.

In block B3, at least one operating parameter of the boiler 1 is optimized based on the validated sensor signals Sv of the respective sensors 20.

In block B4, the overall sensor health confidence values Vo of the respective sensors 20 indicate whether the at least one sensor 20 is faulty.

In one embodiment, the combustion optimization method of the present invention may further comprise the step B5. In block B5, when the overall sensor health confidence value Vo of at least one sensor 20 indicates that the at least one sensor 20 is faulty, a fault warning signal Sf is generated to a graphical user interface 5 (as shown in FIG. 1).

In another embodiment, the combustion optimization method of the present invention may further comprise the step B6. In block B6, when the overall sensor health confidence value Vo of at least one sensor 20 indicates that the at least one sensor 20 is faulty, a repairing command Cr, if applicable, is generated to a sensor controller 6 (as shown in FIG. 1).

The combustion optimization method of the present invention can generate the validated sensor signals Sv of the respective sensors 20 based on the plurality of received sensor signals S and the pre-determined correlations among the plurality of received sensor signals S regardless of the healthy sensors 20 or the faulty sensors 20, so the combustion optimization method of the present invention can reduce erroneous operation, improve service factor, optimize the combustion strategy of the system, increase system robustness and reduce economic loss due to sensor fault.

While the disclosure has been illustrated and described in typical embodiments, it is not intended to be limited to the details shown, since various modifications and substitutions can be made without departing in any way from the spirit of the present disclosure. As such, further modifications and equivalents of the disclosure herein disclosed may occur to persons skilled in the art using no more than routine experimentation, and all such modifications and equivalents are believed to be within the spirit and scope of the disclosure as defined by the following claims.

## Claims

1. A combustion optimization system, comprising:
a boiler (1) having a plurality of zonal locations (10);
a sensor grid (2) comprising a plurality of sensors (20), wherein the plurality of sensors (20) are configured to provide a plurality of sensor signals and the plurality of sensor signals are indicative of measurements of the respective zonal locations (10);
a sensor validation device (3) for receiving the plurality of sensor signals from the plurality of sensors (20) and generating validated sensor signals of the respective sensors (20) based on the plurality of received sensor signals and pre-determined correlations among the plurality of received sensor signals; and
an optimizing controller (4) for optimizing at least one operating parameter of the boiler (1) based on the validated sensor signals of the respective sensors (20).

2. The combustion optimization system of claim 1, wherein the sensor validation device (3) comprises:
an estimation module (31) for receiving the plurality of sensor signals and generating estimated sensor signals of the respective sensors (20), wherein the sensor (3) validation device generates the validated sensor signals based on the respective received sensor signals and the respective estimated sensor signals.

3. The combustion optimization system of claim 2, wherein the estimation module (31) generates the estimated sensor signals based on the pre-determined correlations among the plurality of received sensor signals, the pre-determined correlations comprising spatial correlations among the plurality of received sensor signals.

4. The combustion optimization system of claim 2 or 3, wherein the sensor validation device (3) further comprises:
a diagnosis module (32) for receiving the plurality of sensor signals and determining overall sensor health confidence values (Vo1) of the respective sensors (20) which are indicative of reliability of the respective sensors (20), and
a validation module (330) for generating the validated sensor signals based on the respective received sensor signals, the respective estimated sensor signals and the respective overall sensor health confidence values.

5. The combustion optimization system of claim 4, wherein the estimation module (31) further generates an estimation module confidence value (Ve) which is indicative of reliability of the estimated sensor signals, and wherein the validation module (332) generates the validated sensor signals based on the respective received sensor signals, the respective estimated sensor signals, the respective overall sensor health confidence values (Vo1) and the estimation module confidence value (Ve).

6. The combustion optimization system of claim 4 or 5, wherein the diagnosis module (32) comprises:
a detection module (340) for receiving the plurality of sensor signals, detecting fault types of the respective sensors and generating fault type confidence values of the respective sensors, wherein the fault type confidence values are indicative of fault levels of the respective fault types; and
a fusion module (350) for fusing the generated fault type confidence values of the respective sensors (20) to generate the overall sensor health confidence values of the respective sensors (20).

7. The combustion optimization system of claim 6, wherein the diagnosis module (32) further comprises:
a correlation-conformance module (360) for receiving the plurality of sensor signals and generating correlation-conformance indexes of the respective sensors (20) based on the pre-determined correlations among the plurality of received sensor signals, wherein the correlation-conformance indexes of the respective sensors (20) are indicative of fault levels of the respective sensors (20), and
wherein the fusion module (350) fuses the generated fault type confidence values of the respective sensors (20) and the correlation-conformance indexes of the respective sensors (20) to generate the overall sensor health confidence values of the respective sensors (20).

8. The combustion optimization system of claim 7, wherein the pre-determined correlations among the plurality of received sensor signals comprise spatial correlations among the plurality of received sensor signals.

9. The combustion optimization system of claim 7 or 8, wherein the plurality of sensors (20) comprise a plurality of CO sensors (20) for providing a plurality of CO sensor signals (S1) and a plurality of O₂ sensors (20) for providing a plurality of O₂ sensor signals (S2), and wherein
the estimation module (31) is configured for respectively receiving the plurality of CO sensor signals (S1) and the plurality of O₂ sensor signals (S2), and generating estimated CO sensor signals of the respective CO sensors (20) based on the plurality of received CO sensor signals (S1) and estimated O₂ sensor signals of the respective O₂ sensors (20) based on the plurality of received O₂ sensor signals (S2);
the detection module (340) comprises a CO detection module (341) for receiving the plurality of CO sensor signals (S1), detecting fault types of the respective CO sensors (20) and generating fault type confidence values of the respective CO sensors (20) and a O₂ detection module (342) for receiving the plurality of O₂ sensor signals (S2), detecting fault types of the respective O₂ sensors (20) and generating fault type confidence values of the respective O₂ sensors (20);
the correlation-conformance module (360) is configured for respectively receiving the plurality of CO sensor signals (S1) and the plurality of O₂ sensor signals (S2), and generating CO correlation-conformance indexes of the respective CO sensors (20) and O₂ correlation-conformance indexes of the respective O₂ sensors (20) based on the pre-determined correlations, the pre-determined correlations comprising correlations of physical characteristics between the respective CO sensor signals (S1) and the respective O₂ sensor signals (S2);
the fusion module (350) comprises a CO fusion module (351) for fusing the generated fault type confidence values of the respective CO sensors (20) and the CO correlation-conformance indexes of the respective CO sensors (20) to generate overall CO sensor health confidence values of the respective CO sensor and a O₂ fusion module (352) for fusing the generated fault type confidence values of the respective O₂ sensors (20) and the O₂ correlation-conformance indexes of the respective O₂ sensors (20) to generate overall O₂ sensor health confidence values of the respective O₂ sensors (20); and
the validation module (330) comprises a CO validation module (331) for generating validated CO sensor signals of the respective CO sensors based on the respective received CO sensor signals, the respective estimated CO sensor signals, and the respective overall CO sensor health confidence values, and a O₂ validation module (332) for generating validated O₂ sensor signals of the respective O₂ sensors based on the respective received O₂ sensor signals, the respective estimated O₂ sensor signals, and the respective overall O₂ sensor health confidence values.

10. The combustion optimization system of claim 9, wherein the estimation module (31) further generates an estimation module confidence value which is indicative of reliability of the estimated CO and O₂ sensor signals, and wherein the CO validation module (331) generates the validated CO sensor signals based on the respective received CO sensor signals, the respective estimated CO sensor signals, the respective overall CO sensor health confidence values and the estimation module confidence value, and the O₂ validation module (332) generates the validated O₂ sensor signals based on the respective received O₂ sensor signals, the respective estimated O₂ sensor signals, the respective overall O₂ sensor health confidence values and the estimation module confidence value.

11. A combustion optimization method, comprising:
receiving a plurality of sensor signals (S) from a sensor grid (2), wherein the sensor grid (2) comprises a plurality of sensors (20) which are configured to be in communication with a plurality of zonal locations (10) in a boiler (1);
generating validated sensor signals (Sv) of the respective sensors (20) based on the plurality of received sensor signals (S) and pre-determined correlations among the plurality of received sensor signals (S); and
optimizing at least one operating parameter of the boiler (1) based on the validated sensor signals (Sv) of the respective sensors (20).

12. The combustion optimization method of claim 11, wherein generating the validated sensor signals (Sv) comprises:
generating estimated sensor signals (Se) of the respective sensors (20) by an estimation module (31) based on the plurality of received sensor signals (S);
determining overall sensor health confidence values of the respective sensors (20) based on the plurality of received sensor signals (S), wherein the overall sensor health confidence values of the respective sensors (20) are indicative of reliability of the respective sensors (20); and
generating the validated sensor signals (Sv) based on the respective received sensor signals (S), the respective estimated sensor signals (Se), and the respective overall sensor health confidence values.

13. The combustion optimization method of claim 12, wherein generating the validated sensor signals (Sv) further comprises:
generating an estimation module confidence value, wherein the estimation module confidence value is indicative of reliability of the estimated sensor signals (Se), and wherein generating the validated sensor signals (Sv) comprises: generating the validated sensor signals (Sv) based on the respective received sensor signals (S), the respective estimated sensor signals (Se), the respective overall sensor health confidence values and the estimation module confidence value.

14. The combustion optimization method of claim 12 or 13, wherein generating the estimated sensor signals (Se) comprises generating the estimated sensor signals (Se) based on the pre-determined correlations among the plurality of received sensor signals (S), the pre-determined correlations comprising spatial correlations among the plurality of received sensor signals (S).

15. The combustion optimization method of any of claims 12 to 14, further comprising at least one of;
generating a fault warning signal to a graphical user interface (5) when the overall sensor health confidence value of at least one sensor (20) indicates that the at least one sensor (20) is faulty; and
generating a repairing command to a sensor controller (6) when the overall sensor health confidence value of at least one sensor (20) indicates that the at least one sensor (20) is faulty.
